# EUROPÄISCHE PATENTSCHRIFT

(11) **EP 0 557 483 B1**
(45) Veröffentlichungstag und Bekanntmachung des Hinweises auf die Patenterteilung: **20.11.1996**
(21) Anmeldenummer: 92918784.7
(22) Anmeldetag: 11.09.1992
(51) Int. Cl.: G01R 35/00, G01N 27/22, G01N 33/36

(54) **VERFAHREN UND VORRICHTUNG ZUR KALIBRIERUNG VON PRÜFGERÄTEN FÜR LANGGESTRECKTE TEXTILE MATERIALIEN**
PROCESS AND DEVICE FOR CALIBRATING INSPECTION APPARATUS FOR LONG STRETCHEN-OUT TEXTILE MATERIALS
PROCEDE ET DISPOSITIF D'ETALONNAGE D'APPAREILS DE CONTROLE POUR MATIERES TEXTILES TRES ALLONGEES

(30) Priorität: 18.09.1991 CH 2759/91
(43) Veröffentlichungstag der Anmeldung: 01.09.1993
(73) Patentinhaber: ZELLWEGER LUWA AG, CH-8610 Uster (CH)
(72) Erfinder: ISOTTON, Walter, CH-8610 Uster (CH); ROOS, Gerold, CH-8610 Uster (CH)
(74) Vertreter: Dittrich, Horst
(86) Internationale Anmeldenummer: CH9200187
(87) Internationale Veröffentlichungsnummer: WO9306498

(56) Entgegenhaltungen:
- US-A- 4 208 625

## Beschreibung

Die vorliegende Erfindung betrifft ein Verfahren zur Kalibrierung von Prüfgeräten für langgestreckte textile Materialien als Prüfgut, mit einem Sensor, der ein vom Prüfgut durchlaufenes Messfeld aufweist und ein für die Masse oder den Durchmesser des Prüfguts repräsentatives Signal erzeugt, aus welchem in einer Auswertestufe die Qualität des Prüfguts kennzeichnende Parameter abgeleitet werden.

Derartige Prüfgeräte sind allgemein bekannt und werden beispielsweise von der Anmelderin der vorliegenden Patentanmeldung unter der Bezeichnung USTER TESTER weltweit vertrieben (USTER - eingetragenes Warenzeichen der Zellweger Uster AG). Der verwendete Sensor ist dabei in der Regel ein sogenanntes kapazitives Messorgan, bei dem das Messfeld zwischen zwei Kondensatorplatten gebildet ist und die Masseschwankungen des Prüfguts gemessen werden. Von diesen Masseschwankungen kann eine Reihe qualitätsbestimmender Parameter, wie beispielsweise die Ungleichmässigkeit, der Variationskoeffizient, die relative Feinheit oder Nummer und andere mehr abgeleitet werden.

Andere bekannte Sensoren tasten das Prüfgut optisch ab und messen die Durchmesserschwankungen des Prüfguts, von denen ebenfalls Parameter der genannten Art ableitbar sind.

Eine wesentliche Anforderung an Prüfgeräten dieser Art ist die Möglichkeit der Ueberprüfung der Linearität des Sensorsignals über den gesamten Messbereich und der Signalauswertung. Zur Ueberprüfung der Signalauswertung verfügt der USTER TESTER über interne Gerätetests, die redundant ausgelegt sind und insbesondere auch die verwendeten Referenzquellen überprüfen und messen. Eine Ueberprüfung der Linearität des Sensors könnte bisher höchstens mit Hilfe von "Eichgarnen" erfolgen, was aber sehr umständlich wäre.

Durch die vorliegende Erfindung soll nun ein Verfahren angegeben werden, durch dessen Anwendung die Linearität und der Frequenzgang des Sensors überprüft werden können. Dieses Verfahren soll vorzugsweise dynamischer Art sein und es soll eine sichere, einfache und rasche Ueberprüfung der Linearität über den gesamten Messbereich ermöglichen.

Die gestellte Aufgabe wird erfindungsgemäss dadurch gelöst, dass im Messfeld durch periodische Bewegung eines ersten Testorgans ein Wechselsignal erzeugt wird, welches durch ein stufenweise in das Messfeld einbringbares zweites Testorgan über den Messbereich verschoben wird, und dass bei verschiedenen Punkten des Messbereichs die Standardabweichung des Wechselsignals überprüft wird.

Die Erfindung betrifft weiter eine Vorrichtung zur Durchführung des genannten Verfahrens, mit wahlweise im Messfeld positionierbaren Mitteln zur Erzeugung von Sensorsignalen an verschiedenen Punkten des Messbereichs.

Die erfindungsgemässe Vorrichtung ist dadurch gekennzeichnet, dass die genannten Mittel durch ein erstes und ein zweites Testorgan gebildet sind, wobei das erste Testorgan antreibbar ausgebildet und zur Erzeugung eines Wechselsignals vorgesehen und das zweite Testorgan stufenweise in das Messfeld bewegbar und zur Verschiebung des Wechselsignals an verschiedene Punkte des Messbereichs vorgesehen ist.

Im folgenden wird die Erfindung anhand eines Ausführungsbeispiels und der Zeichnungen näher erläutert; es zeigt:
- Fig. 1: eine Aufrissdarstellung einer erfindunsgemässen Testvorrichtung, in Richtung zum Sensor eines Prüfgeräts hin gesehen,
- Fig. 2: einen Schnitt nach der Linie II-II von Fig. 1,
- Fig. 3: einen Schnitt nach der Linie III-III von Fig. 1,
- Fig. 4: ein erstes Detail der Testvorrichtung,
- Fig. 5a,b: ein zweites Detail der Testvorrichtung in zwei Ansichten,
- Fig. 6a,b: ein drittes Detail der Testvorrichtung in zwei Ansichten; und
- Fig. 7: ein Diagramm zur Erläuterung des Funktionsablaufs.

Das Prinzip des erfindungsgemässen Testverfahrens besteht darin, mit Eichlehren an mehreren Punkten des Messbereichs ein Wechselsignal mit konstanter Amplitude zu erzeugen und die Standardabweichung an den genannten Punkten zu messen. Um die geforderte Linearität zu gewährleisten, muss bei konstanter Amplitude die Standardabweichung ebenfalls konstant sein oder jedenfalls innerhalb vorgegebener Toleranzen liegen. Anstatt mehrerer Eichlehren wird ein einziges Testorgan verwendet, welches ein Wechselsignal erzeugt. Dieses Wechselsignal, welches naturgemäss eine konstante Amplitude aufweist, wird durch ein zweites Testorgan, welches stufenweise in das Messfeld des Sensors einbringbar ist und damit dem Vorhandensein von Prüfgut verschiedener Masse oder verschiedenen Durchmessers im Messfeld des Sensors entspricht, an verschiedene Punkte des Messbereichs geschoben. Damit wird an verschiedenen Punkten des Messbereichs entsprechend mehreren Eichlehren mit verschiedenen Massen oder Durchmessern ein Signal konstanter Amplitude erhalten und die Linearität des Sensors kann anhand der Standardabweichung des Signals überprüft werden.

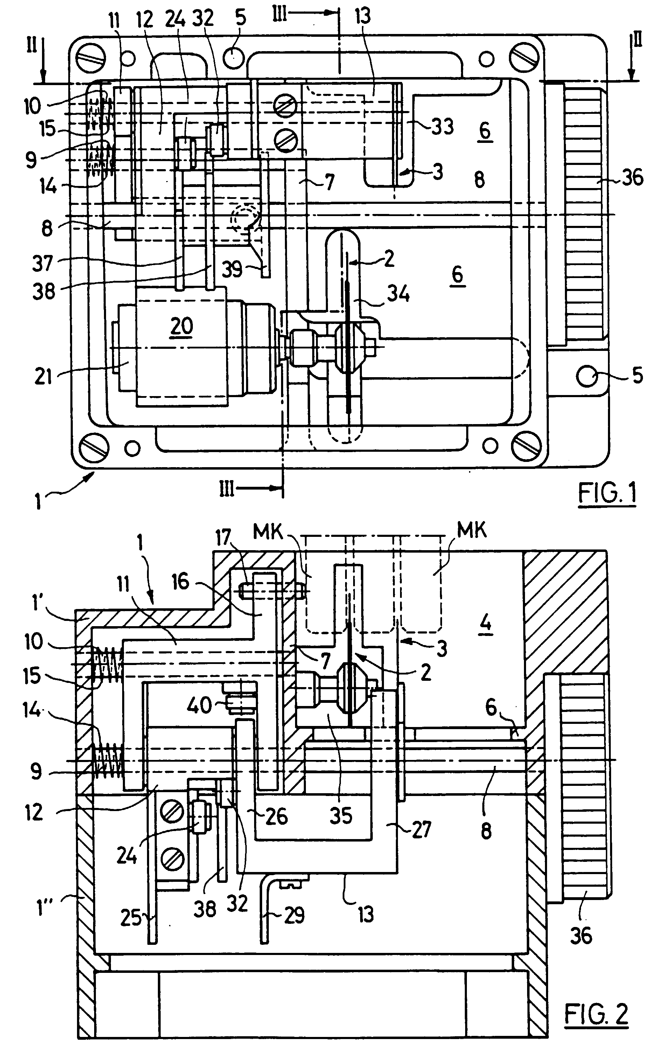

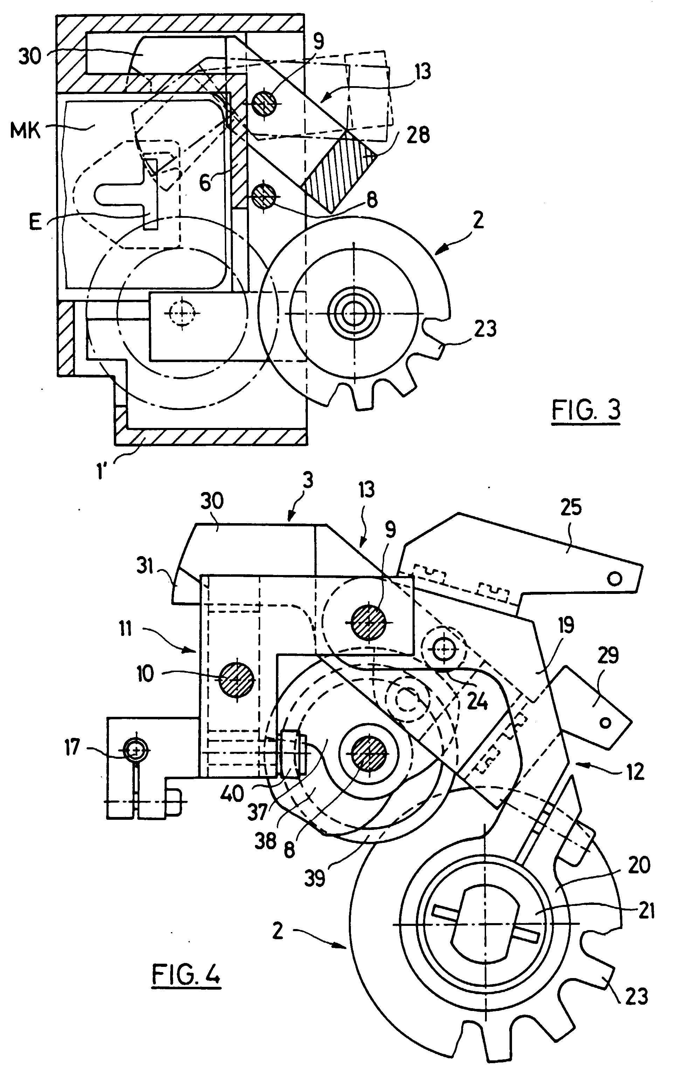

Gemäss den Fig. 1 bis 4 besteht die Testvorrichtung im wesentlichen aus einem Gehäuse 1, welches im Inneren zwei Testorgane, und zwar ein Impulsrad 2 und eine Zusatzmasse 3, an seiner der Bedienungsperson zugewandten Vorderseite einen abnehmbaren Deckel (nicht eingezeichnet) und an seiner dem zu überprüfenden Prüfgerät zugewandten Rückseite eine gegen die Rückseite hin offene Messkammer 4 aufweist. Die dargestellte Testvorrichtung ist zur Ueberprüfung eines als USTER TESTER (siehe dazu beispielsweise die CH-Patente 671 105, 671 980 bis 671 983) bekannten Garnprüfgeräts vorgesehen, welches bekanntlich einen Messkamm für das Prüfgut aufweist, dessen plattenartige Vorsprünge MK (Fig. 2) Messspalte verschiedener Spaltbreite begrenzen. Jeder Vorsprung MK enthält eine als Kondensatorplatte wirkende Elektrode E (Fig. 3). Der Messkamm, der eine prismatische Form aufweist, ist von einem rahmenartigen Fadenführungsblock umgeben, welcher mittels zweier Zentrierstifte relativ zum Messkamm positioniert und an das Prüfgerät angeschraubt ist.

Zum Aufsetzen der Testvorrichtung wird der Fadenführungsblock abgenommen und die Testvorrichtung wird auf die Zentrierstifte geschoben und mit zwei das Gehäuse 1 durchstossenden Schraubbolzen, die in Bohrungen 5 geführt sind, am Prüfgerät fixiert. In dieser Position nehmen die Vorsprünge MK des Messkamms die in Fig. 3 eingezeichnete Stellung ein und ragen über ihre ganze Tiefe in die in diesem Stadium ansonsten leere Messkammer 4. In Fig. 2 ragen die gestrichelt eingezeichneten Vorsprünge MK der besseren Uebersichtlichkeit halber nicht vollständig in die Messkammer 4. Das Gehäuse 1 besteht aus zwei miteinander verschraubten Teilen 1' und 1", von denen der eine Teil 1' im wesentlichen die Messkammer 4 und der andere Teil 1" die erforderlichen elektronische Schaltung enthält. Die beiden Testorgane 2 und 3 sind zwischen den beiden Gehäuseteilen verstellbar. In Fig. 3 ist nur der die Messkammer 4 enthaltende Gehäuseteil 1' dargestellt.

Die Messkammer 4 ist gegen den Gehäuseteil 1" hin durch eine Rückwand 6 abgeschlossen; die in den Fig. 1 und 2 linke Seitenwand der Messkammer 4 setzt sich nach vorne fort und bildet einen Quersteg 7. In diesem Quersteg ist eine über die ganze Breite des Gehäuses 1 verlaufende Antriebswelle 8 sowie eine erste Lagerwelle 9 gelagert. Eine zweite Lagerwelle 10 ist seitlich neben der Messkammer 4 vorgesehen.

Wie den Fig. 1 und 2 zu entnehmen ist, sind auf der ersten Lagerwelle 9 nebeneinander eine Schiebegabel 11, ein das Impulsrad 2 tragender erster Schwenkhebel 12 und ein die Zusatzmasse 3 tragender zweiter Schwenkhebel 13 gelagert. Die Schiebegabel 11 und die beiden Schwenkhebel 12 und 13 sind als Block gemeinsam in Längsrichtung der Lagerwelle 9 verschiebbar, wobei dieser Block gegen die linke Gehäusewand durch eine Druckfeder 14 abgestützt ist. Die Schiebegabel 11 ist zusätzlich auch auf der zweiten Lagerwelle 10 gelagert, wobei auch hier eine zwischen der linken Gehäusewand und der Schiebegabel 11 wirkende Druckfeder 15 vorgesehen ist.

Die Schiebegabel 11 dient zur Feinpositionierung von Impulsrad 2 und Zusatzmasse 3 relativ zu den Messspalten zwischen den Vorsprüngen MK des Messkamms (Fig. 2). Zu diesem Zweck weist die Schiebegabel 11 einen seitlich neben der Messkammer 4 bis nahe an deren offene Wand ragenden Fortsatz 16 auf, auf welchem ein in einer Oeffnung in der linken Seitenwand der Messkammer 4 geführter Anschlagbolzen 17 montiert ist. Dieser ist in seiner den Fortsatz 16 überragenden Höhe einstellbar. Wenn die Testvorrichtung über den Messkamm geschoben und am Prüfgerät fixiert ist, befindet sich der aus der Schiebegabel 11 und den beiden Schwenkhebeln 12 und 13 gebildete Block in Ruhestellung (Impulsrad 2 und Zusatzmasse 3 ausserhalb der Messkammer 4 in der in Fig. 3 voll ausgezogenen Stellung), in der der Anschlagbolzen 17 die ihn führende Seitenwand der Messkammer 4 nicht überragt. Anschliessend wird die Schiebegabel 11 bezüglich Fig. 1 und 2 nach rechts verschoben, und zwar solange, bis der Anschlagbolzen 17 an der äusseren Seitenwand des ersten Messkammvorsprungs MK in Anschlag kommt. Bei dieser in Fig. 2 angedeuteten Position fluchten das Impulsrad 2 und die Zusatzmasse 3 mit ihren Messspalten und können in diese eingefahren werden.

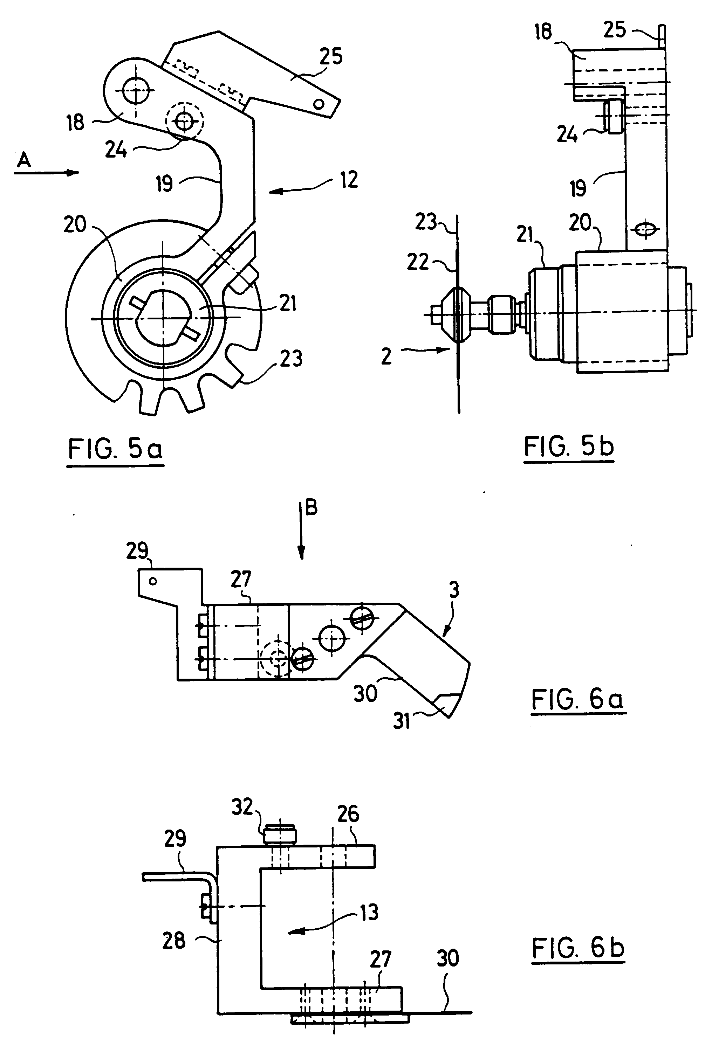

Der erste Schwenkhebel 12, der in den Fig. 5a und 5b im Detail dargestellt ist, und zwar in Fig. 5a in Richtung des Pfeiles III von Fig. 1 und in Fig. 5b in Richtung des Pfeiles A von Fig. 5a, besteht aus einem auf der ersten Lagerwelle 9 gelagerten buchsenartigen Lagerteil 18, aus einem Verbindungsteil 19 und aus einem klauenartigen Einspannteil 20. Im letzteren ist ein Minimotor 21 eingespannt, auf dessen Antriebswelle das Impulsrad 2 montiert ist. Das Impulsrad 2 besteht aus einer Stützscheibe 22 und aus einer mit dieser verklebten dünnen Impulsscheibe 23 aus einem ausreichend steifen Material und einer geringen Dielektrizitätskonstante, beispielsweise aus Plexiglas oder Teflon. Am Umfang des Impulsrades 2 ist eine geeignete Verzahnung vorgesehen.

Im Uebergangsbereich zwischen Lagerteil 18 und Verbindungsteil 19 des ersten Schwenkhebels 12 ist eine Laufrolle 24 vorgesehen, welche auf einer Kurvenscheibe umläuft, wodurch der erste Schwenkhebel 12 in die in Fig. 3 strichpunktiert eingezeichnete Arbeitslage verschwenkbar ist. In dieser ragt der verzahnte Kranz der Impulsscheibe 23 gerade in den Messbereich der Elektroden E der Messkammervorsprünge MK, so dass bei Rotation der Impulsscheibe 23 im Messkamm ein Wechselsignal erzeugt wird. Im Bereich des Lagerteils 18 ist ausserdem eine Lasche 25 befestigt, an welcher das eine Ende einer Zugfeder (nicht dargestellt) angreift, deren anderes Ende im Bereich der dem Betrachter in Fig. 1 zugewandten Stirnseite des Bodens des Gehäuses 1 verankert ist und dadurch die Lasche 25 nach unten zieht, wodurch der 1. Schwenkhebel 12 mit Federspannung in seine Arbeitsstellung gedrückt ist. Die Ueberprüfung des Frequenzgangs des Sensors erfolgt durch Einstellung verschiedener Drehzahlen des Minimotors 21 und damit der Impulsscheibe 23.

Der zweite Schwenkhebel 13, der in den Fig. 6a und 6b im Detail dargestellt ist, und zwar in Fig. 6a entgegen der Richtung des Pfeiles III von Fig. 1 und in Fig. 6b in Richtung des Pfeiles III von Fig. 1 und in Fig. 6b in Richtung des Pfeiles B von Fig. 6a, ist gabelartig ausgebildet und besteht aus zwei Seitenarmen 26, 27 und aus einem dieser verbindenden Mittelteil 28. Die beiden Seitenarme 26, 27 sind auf der ersten Lagerwelle 9 gelagert und am Mittelteil 28 ist eine Lasche 29 befestigt, an welcher in gleicher Art wie an der Lasche 25 des ersten Schwenkhebels 12 eine Zugfeder (nicht dargestellt) angreift, welche die Lasche 29 nach unten zieht.

Am Seitenteil 27 ist ein Flügel 30 aus dem qleichen Material wie die Impulsscheibe 23 montiert, welcher an seinem freien Ende einen Bereich 31 von geringerer Dicke aufweist. Der Flügel 30 dient als in einen Messspalt einschwenkbare Zusatzmasse, welche stufenweise verschieden tief in den Messspalt einschwenkbar ist, wodurch verschieden grosse Massen in diesen gelangen. In Fig. 3 ist die Ruhestellung des zweiten Schwenkhebels 13 mit vollen Linien eingezeichnet, die erste Arbeitsstellung, in der nur der Bereich 31 in den Bereich der Elektroden E geschwenkt ist, strichpunktiert, und die Endstellung, in der der an Bereich 31 anschliessende dickere Flügelbereich in den Bereich der Elektroden E geschwenkt ist, gestrichelt. Alternativ kann der Bereich 31 in der ersten Arbeitsstellung nur teilweise und in der zweiten Arbeitsstellung vollständig in den Bereich der Elektroden E geschwenkt werden. Als Antriebsmittel für die Verstellung des zweiten Schwenkhebels 13 dient eine am Seitenarm 26 montierte Laufrolle 32, die auf einer Kurvenscheibe umläuft. Wie sich den Figuren 1 bis 3 entnehmen lässt, weisen die Rückwand 6 und der Boden der Messkammer 4 Durchbrechungen 33 bis 35 für den Durchtritt des Impulsrades 2 und der Zusatzmasse 3 in die Messkammer 4 auf.

Die schon erwähnten Nockenscheiben für den Schwenkantrieb der beiden Schwenkhebel 12 und 13 sind auf der Antriebswelle 8 (Fig. 1) montiert, welche mit einem aussen am Gehäuse 1 angeordneten Handrad 36 verbunden ist. In Fig. 1 ist auf der Antriebswelle 8 eine Kurvenscheibe 37 für die Laufrolle 24, eine Kurvenscheibe 38 für die Laufrolle 32 sowie eine Nockenscheibe 39 mit einem seitlich abstehenden Steuernocken erkennbar. Letztere dient zur Verschiebung der Schiebegabel 11, welche mit einer an der Nockenscheibe 39 seitlich anliegenden Laufrolle 40 versehen ist. Das Handrad 36 ist durch eine Rastvorrichtung (nicht dargestellt) in insgesamt vier Positionen fixierbar: In einer Ruheposition entsprechend der in Fig. 3 mit vollen Linien dargestellten Lage der beiden Schwenkhebel 12 und 13, in einer ersten Arbeitsposition mit eingeschwenkter Impulsscheibe 23, in einer zweiten Arbeitsposition mit zusätzlich eingeschwenktem Bereich 31 des Flügels 30 und in einer dritten Arbeitsposition mit zusätzlich voll eingeschwenktem Flügel 30. Die dritte Arbeitsposition ist auch in den Fig. 1 und 2 dargestellt.

In Fig. 4 ist der genannte, aus der Schiebegabel 11 und aus den beiden Schwenkhebeln 12 und 13 gebildete Block zusammen mit den auf der Antriebswelle 8 befestigten Kurvenscheiben 37 und 38 und der Nockenscheibe 39 in einen vergrösserten Massstab dargestellt, und zwar bezogen auf Fig. 1 von links gesehen.

Die Kurvenscheibe 37 weist einen grossen und einen kleinen Durchmesser und einen diese verbindenden Uebergangsteil auf, die Kurvenscheibe 38 ebenfalls einen grossen und einen kleinen Durchmesser sowie einen mittleren Durchmesser (der nur wenig kleiner ist als der grosse) und zwei Uebergangsteile. Entsprechend dieser Ausbildung der Kurvenscheiben erfolgt die Verstellung von der dritten Arbeitsposition in die Ruheposition durch Zurückdrehen des Handrads 36 (Fig. 1).

Im dargestellten Momentanzustand ist der genannte Block via Schiebegabel 11 und Anschlagbolzen 17 relativ zum Messkamm MK positioniert; die Laufrolle 24 läuft auf dem grossen Durchmesser der Kurvenscheibe 37 und die Laufrolle 32 auf dem kleinen Durchmesser der Kurvenscheibe 38. Bei einer Drehung der Antriebswelle 8 mit dem Kurvenscheibenpaket im Gegenuhrzeigersinn wird nach etwa 90° die Laufrolle 24 auf den kleinen Durchmesser der Kurvenscheibe 37 geführt, wodurch der erste Schwenkhebel 12 in seine Arbeitsstellung (erste Arbeitsposition des Handrads 36) gelangt. Die Laufrolle 32 erreicht nach knapp 180° den mittleren und nach weiteren 20° den grossen Durchmesser, wodurch der zweite Schwenkhebel 13 in seine beiden Arbeitsstellungen (zweite und dritte Arbeitsposition des Handrads 36) gelangt.

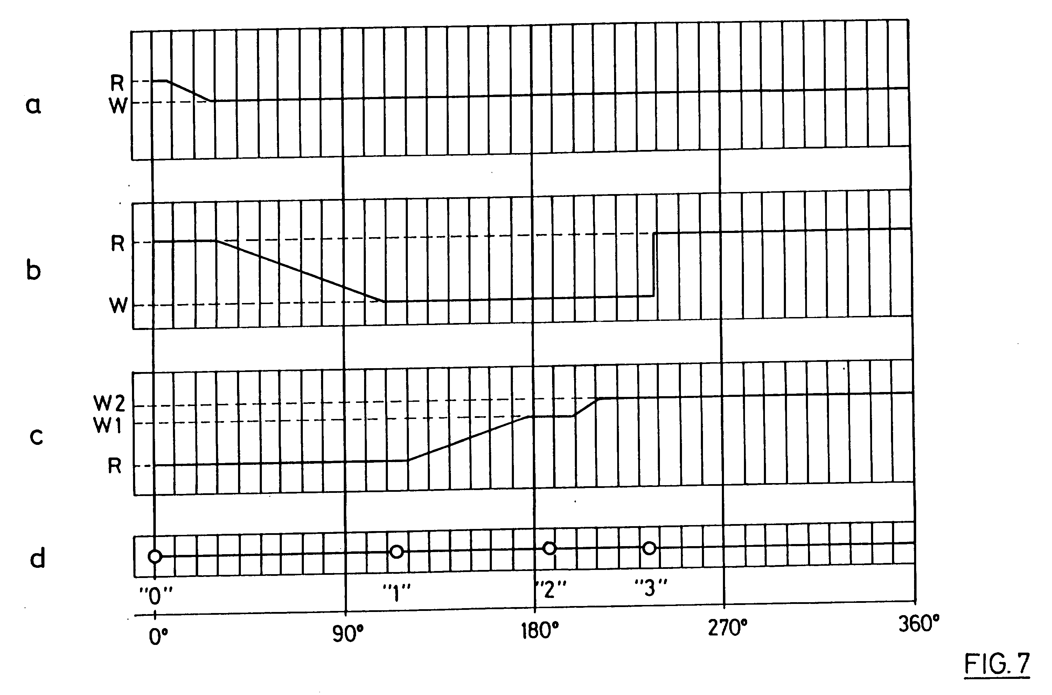

Fig. 7 zeigt ein Diagramm des Steuerungsablaufs des in Fig. 4 dargestellten Blocks über einen Zyklus von 360°, wobei in den einzelnen Zeilen mit R die Ruhestellung und mit W die Arbeitsstellung des jeweiligen Verstellorgans bezeichnet ist. Zeile a zeigt die Verschiebung der Schiebegabel 11, Zeile b die Verschwenkung des ersten Schwenkhebels 12 mit dem Impulsrad 2, Zeile c die Verschwenkung des zweiten Schwenkhebels 13 und der Zusatzmasse 3 (zwei Arbeitsstellungen W1 und W2 entsprechend der zweiten und dritten Arbeitsposition des Handrads 36) und Zeile d zeigt die entsprechenden Ruhepositionen des Handrads 36, und zwar Rastposition "0" bei 0° entsprechend der Ruheposition, Rastposition "1" bei 115° entsprechend der ersten Arbeitsposition, Rastposition "2" bei 186° entsprechend der zweiten Arbeitsposition und Rastposition "3" bei 235° entsprechend der dritten Arbeitsposition. Wie schon erwähnt wurde, erfolgt die Verstellung des Handrads 36 von Rastposition "3" in Rastposition "0" durch Zurückdrehen. Die Verschiebung der Schiebegabel 11 und damit die Feinpositionierung von Impulsrad 2 und Zusatzmasse 3 erfolgt zwischen 7,5° und 27°, also vor dem Einschwenken des Impulsrads 2 bei 30°.

Wenn auch die Testvorrichtung in Zusammenhang mit einem Prüfgerät mit einem kapazitiven Messorgan beschrieben wurde, so ist für den Fachmann klar, dass das zugrundeliegende Verfahren, gegebenenfalls unter Vornahme geringfügiger Anpassungen, auch bei optischen oder elektrooptischen Messorganen verwendet werden kann.

## Patentansprüche

1. Verfahren zur Kalibrierung von Prüfgeräten für langgestreckte textile Materialien als Prüfgut, mit einem Sensor (MK), der ein vom Prüfgut durchlaufenes Messfeld aufweist und ein für die Masse oder den Durchmesser des Prüfguts repräsentatives Signal erzeugt, aus welchem in einer Auswertestufe die Qualität des Prüfguts kennzeichnende Parameter abgeleitet werden, dadurch gekennzeichnet, dass im Messfeld durch periodische Bewegung eines ersten Testorgans (2) ein Wechselsignal erzeugt wird, welches durch ein stufenweise in das Messfeld einbringbares zweites Testorgan (3) über den Messbereich verschoben wird, und dass bei verschiedenen Punkten des Messbereichs die Standardabweichung des Wechselsignals überprüft wird.

2. Verfahren nach Anspruch 1, dadurch gekennzeichnet, dass das erste Testorgan (2) durch eine rotierbare, zur Erzeugung von Impulsen ausgebildete Scheibe (23) und das zweite Testorgan (3) durch einen messerartigen Flügel (30, 31) gebildet ist, welcher schrittweise in das Messfeld bewegt wird.

3. Verfahren nach Anspruch 2, dadurch gekennzeichnet, dass bei Prüfgeräten mit einem mehrere Messschlitze für das Prüfgut aufweisenden kapazitiven Sensor (MK) das erste und das zweite Testorgan (2 bzw. 3) in verschiedenen Messschlitzen positioniert werden, wobei in einem ersten Schritt das erste Testorgan (2) und in folgenden Schritten das zweite Testorgan (3) in den zugeordneten Messschlitz bewegt wird.

4. Vorrichtung zur Durchführung des Verfahrens nach Anspruch 1, mit im Messfeld positionierbaren Mitteln zur Erzeugung von Sensorsignalen an verschiedenen Punkten des Messbereichs, dadurch gekennzeichnet, dass die genannten Mittel durch ein erstes und ein zweites Testorgan (2,3) gebildet sind, wobei das erste Testorgan (2) antreibbar ausgebildet und zur Erzeugung eines Wechselsignals vorgesehen und das zweite Testorgan (3) stufenweise in das Messfeld bewegbar und zur Verschiebung des Wechselsignals an verschiedene Punkte des Messbereichs vorgesehen ist.

5. Vorrichtung nach Anspruch 4, gekennzeichnet durch ein auf das Prüfgerät aufsetzbares Gehäuse (1), welches die beiden Testorgane (2, 3) enthält.

6. Vorrichtung nach Anspruch 4 oder 5, dadurch gekennzeichnet, dass das erste Testorgan (2) durch eine rotierbare Impulsscheibe (23) und das zweite Testorgan (3) durch einen messerartigen Flügel (30,31) gebildet ist.

7. Vorrichtung nach Anspruch 6, dadurch gekennzeichnet, dass die Impulsscheibe (23) durch ein am Umfang mit gleichmässig beabstandeten Oeffnungen oder Vorsprüngen, vorzugsweise mit einer Verzahnung, versehenes Rad gebildet ist.

8. Vorrichtung nach den Ansprüchen 5 und 6, dadurch gekennzeichnet, dass das Gehäuse (1) eine auf den Sensor (MK) aufsetzbare Messkammer (4), Positioniermittel (11) zum Ausrichten der Testorgane (2,3) relativ zum Sensor (MK), Verstellmittel (12,13) zur Bewegung der beiden Testorgane in das Messfeld und Antriebsmittel (21) für das erste Testorgan aufweist.

9. Vorrichtung nach Anspruch 8, dadurch gekennzeichnet, dass die genannten Positionier- und Verstellmittel (11;12,13) auf einer quer zum Messfeld orientierten Lagerwelle (9) gelagert und zu einem gemeinsam auf dieser Lagerwelle verschiebbaren Block zusammengefasst sind, und dass die Positioniermittel eine Schiebegabel (11) mit einem gegen eine Aussenfläche des Sensors (MK) gerichteten Anschlagbolzen (17) und ein Antriebsmittel zur Verschiebung längs der Lagerwelle aufweisen.

10. Vorrichtung nach Anspruch 9, dadurch gekennzeichnet, dass die Verstellmittel je einen mit einem Antriebsmittel versehenen Schwenkhebel (12,13) für die beiden Testorgane (2,3) aufweisen, und dass am Schwenkhebel (12) für das erste Testorgan (2) ein Motor (21) für dessen Antrieb angeordnet ist.

11. Vorrichtung nach Anspruch 10, gekennzeichnet durch eine im Gehäuse (1) parallel zur Lagerwelle (9) angeordnete Antriebswelle (8) mit auf dieser getragenen Kurven- und/oder Nockenscheiben (37,38,39), welche zusammen mit entsprechenden Laufrollen (24,32,40) als Antriebsmittel für die Positionier- und Verstellmittel (11;12,13) wirken.

12. Vorrichtung nach Anspruch 11, dadurch gekennzeichnet, dass die Antriebswelle (8) das Gehäuse (1) an einem Ende überragt und an diesem Ende ein Handrad (36) trägt, welches in verschiedenen Positionen einrastbar ist.

13. Vorrichtung nach Anspruch 12, dadurch gekennzeichnet, dass vier Rastpositionen des Handrads (3) vorgesehen sind, und zwar eine Ruheposition "0"; eine erste Arbeitsposition "1", bei der die Impulsscheibe (23) in das Messfeld geschwenkt ist; eine zweite Arbeitsposition "2", bei der zusätzlich zur Impulsscheibe ein erster Bereich (30) des messerartigen Flügels mit kleiner Masse in das Messfeld geschwenkt ist; und eine dritte Arbeitsposition "3", bei der zusätzlich zur Impulsscheibe ein zweiter Bereich (31) des Flügels mit grösserer Masse in das Messfeld geschwenkt ist.

14. Vorrichtung nach einem der Ansprüche 4 bis 13, dadurch gekennzeichnet, dass der Sensor durch ein kapazitives Messorgan (MK) gebildet ist, und dass die Impulsscheibe (23) und der Flügel (30, 31) aus einem Material mit geringer Dielektrizitätskonstante mit einem Wert von vorzugsweise nicht grösser als 2,5 bestehen.

15. Vorrichtung nach einem der Ansprüche 4 bis 13, dadurch gekennzeichnet, dass der Sensor durch ein optisches oder ein elektrooptisches Messorgan gebildet ist.

## Claims

1. Process for calibrating testers for elongated textile materials as the material being tested, having a sensor (MK) which has a measuring field, run through by the material being tested, and generates a signal representative of the mass or the diameter of the material being tested, from which signal parameters characterising the quality of the material being tested can be derived in an evaluation stage, characterised in that an alternating signal is generated in the measuring field by periodic movement of a first test element (2), which alternating signal is displaced over the measuring range by a second test element (3) which can be introduced in stages into the measuring field, and in that the standard deviation of the alternating signal is verified at various points of the measuring range.

2. Process according to Claim 1, characterised in that the first test element (2) is formed by a rotatable disc (23), designed for generating pulses, and the second test element (3) is formed by a knife-like lobe (30, 31), which is moved in steps into the measuring field.

3. Process according to Claim 2, characterised in that, in the case of testers having a capacitive sensor (MK) which has a plurality of measuring slots for the material being tested, the first test element (2) and the second test element (3) are positioned in various measuring slots, the first test element (2) being moved into the assigned measuring slot in a first step and the second test element (3) being moved into the assigned measuring slot in following steps.

4. Apparatus for carrying out the process according to Claim 1, having means which are positionable in the measuring field for generating sensor signals at various points of the measuring range, characterised in that the said means are formed by a first test element (2) and a second test element (3), the first test element (2) being of drivable design and provided for generating an alternating signal and the second test element (3) being movable in stages into the measuring field and provided for displacing the alternating signal to various points of the measuring range.

5. Apparatus according to Claim 4, characterised by a housing (1) which can be fitted onto the tester and contains the two test elements (2, 3).

6. Apparatus according to Claim 4 or 5, characterised in that the first test element (2) is formed by a rotatable pulse disc (23) and the second test element (3) is formed by a knife-like lobe (30, 31).

7. Apparatus according to Claim 6, characterised in that the pulse disc (23) is formed by a wheel provided on the circumference with evenly spaced openings or projections, preferably with a toothing.

8. Apparatus according to Claims 5 and 6, characterised in that the housing (1) has a measuring chamber (4) which can be fitted onto the sensor (MK), positioning means (11) for aligning the test elements (2, 3) in relation to the sensor (MK), adjusting means (12, 13) for moving the two test elements into the measuring field and drive means (21) for the first test element.

9. Apparatus according to Claim 8, characterised in that the said positioning and adjusting means (11; 12, 13) are mounted on a bearing shaft (9) oriented transversely to the measuring field and are combined to form a block displaceable together on this bearing shaft, and in that the positioning means have a sliding fork (11) with a stop bolt (17), directed towards an outside surface of the sensor (MK), and a drive means for displacement along the bearing shaft.

10. Apparatus according to Claim 9, characterised in that the adjusting means each have a pivoted lever (12, 13), provided with a drive means, for the two test elements (2, 3), and in that a motor (21) is arranged on the pivoted lever (12) for the first test element (2) for its drive.

11. Apparatus according to Claim 10, characterised by a drive shaft (8) which is arranged in the housing (1) parallel to the bearing shaft (9) and has cam plates (37, 38, 39) which are borne on the said shaft and interact with corresponding running rollers (24, 32, 40) as drive means for the positioning and adjusting means (11; 12, 13).

12. Apparatus according to Claim 11, characterised in that the drive shaft (8) protrudes beyond the housing (1) at one end and at this end bears a hand wheel (36), which can be detained in various positions.

13. Apparatus according to Claim 12, characterised in that four detaining positions of the hand wheel (3) are provided, to be precise a rest position "0"; a first working position "1", in which the pulse disc (23) has been pivoted into the measuring field; a second working position "2", in which, in addition to the pulse disc, a first region (30) of the knife-like lobe of small mass has been pivoted into the measuring field; and a third working position "3", in which, in addition to the pulse disc, a second region (31) of the lobe of greater mass has been pivoted into the measuring field.

14. Apparatus according to one of Claims 4 to 13, characterised in that the sensor is formed by a capacitive measuring element (MK), and in that the pulse disc (23) and the lobe (30, 31) consist of a material having a low dielectric constant of a value of preferably not greater than 2.5.

15. Apparatus according to one of Claims 4 to 13, characterised in that the sensor is formed by an optical or an electro optical measuring element.

## Revendications

1. Procédé pour l'étalonnage d'appareils d'essai ou de contrôle pour matériaux textiles allongés, en tant que matériaux d'essai, avec un capteur ou détecteur (MK) qui comprend un champ de mesure parcouru par le matériau d'essai et produit un signal représentatif de la masse ou du diamètre du matériau d'essai à partir duquel sont dérivés dans un étage d'analyse les paramètres caractérisant la qualité du matériau d'essai ou de contrôle, caractérisé en ce que l'on produit dans le champ de mesure par déplacement périodique d'un premier organe d'essai ou de contrôle (2) un signal alternatif qui est déplacé sur la zone de mesure par un deuxième organe d'essai (3) susceptible d'être introduit de façon pas-à-pas dans le champ de mesure, et en ce que l'écart standard du signal alternatif est contrôlé dans différents points de la zone de mesure.

2. Procédé selon la revendication 1, caractérisé en ce que le premier organe d'essai (2) est formé par un disque rotatif (23) configuré pour la production d'impulsions et le deuxième organe d'essai (3) est formé par une aile en forme de couteau (30, 31) qui est déplacée de façon pas-à-pas dans le champ de mesure.

3. Procédé selon la revendication 2, caractérisé en ce que, dans le cas d'appareils d'essai de contrôle avec un capteur capacitif (MK) comprenant plusieurs fentes de mesure pour le matériau d'essai, le premier organe d'essai et le deuxième organe d'essai (2 ou 3) sont positionnés dans différentes fentes de mesure, dans un premier pas le premier organe d'essai (2) et dans des pas suivants le deuxième organe d'essai étant déplacé dans la fente de mesure associée.

4. Dispositif pour la mise en oeuvre du procédé selon la revendication 1, avec des moyens susceptibles d'être positionnés dans le champ de mesure pour la production de signaux de capteur dans différents points de la zone de mesure, caractérisé en ce que lesdits moyens sont formés par un premier et un deuxième organes d'essai ou de contrôle (2, 3), le premier organe d'essai (2) étant configuré de façon à pouvoir être entraîné et est prévu pour la production d'un signal alternatif, et le deuxième organe d'essai ou de contrôle étant déplaçable, de façon pas-à-pas, dans le champ de mesure et prévu pour le déplacement du signal alternatif dans différents points de la zone de mesure.

5. Dispositif selon la revendication 4, caractérisé par un boîtier (1) susceptible d'être placé sur un appareil d'essai et qui comprend les deux organes d'essai (2, 3).

6. Dispositif selon la revendication 4 ou 5, caractérisé en ce que le premier organe d'essai (2) est formé par un disque d'impulsion rotatif (23) et le deuxième organe d'essai (3) est formé par une aile en forme de couteau (30, 31).

7. Dispositif selon la revendication 6, caractérisé en ce que le disque d'impulsion (23) est formé par une roue pourvue à sa périphérie d'ouvertures ou de saillies, disposée à des distances régulières, de préférence d'une denture.

8. Dispositif selon la revendication 5 et 6, caractérisé en ce que le boîtier (1) comprend une chambre de mesure (4) susceptible d'être placée sur le capteur (MK), des moyens de positionnement (11) pour l'orientation des organes d'essai (2, 3) par rapport au capteur (MK), des moyens de réglage ou de déplacement (12, 13) pour le déplacement des deux organes d'essai dans le champ de mesure et des moyens d'entraînement (21) pour le premier organe d'essai.

9. Dispositif selon la revendication 8, caractérisé en ce que les moyens précités de positionnement et de réglage ou de déplacement (11 ; 12, 13) sont supportés sur un arbre de support (9) orienté perpendiculairement au champ de mesure et sont réunis pour former un bloc déplaçable en commun sur cet arbre de support, et en ce que les moyens de positionnement comprennent une fourche de coulissement (11) avec un boulon de butée (17) orienté vers la face extérieure du capteur (MK) et un moyen d'entraînement pour le déplacement le long de l'arbre de support.

10. Dispositif selon la revendication 9, caractérisé en ce que les moyens de déplacement ou de réglage comprennent chacun un levier pivotant (12, 13) pourvu de moyens d'entraînement, pour les deux organes d'essai (2, 3), et en ce que sur le levier pivotant (12) pour le premier organe d'essai (2) est disposé un moteur (21) pour l'entraînement de celui-ci.

11. Dispositif selon la revendication 10, caractérisé par un arbre d'entraînement (8) disposé dans le boîtier (1), parallèlement à l'arbre de support (9) et comprenant des disques à courbe et/ou à came (37, 38, 39) portés sur celui-ci, qui, ensemble avec des galets de roulement correspondants (24, 32, 40) agissent comme moyen d'entraînement des moyens de positionnement et de déplacement (11 ; 12, 13).

12. Dispositif selon la revendication 11, caractérisé en ce que l'arbre d'entraînement (8) fait saillie du boîtier (1) à une extrémité et porte à cette extrémité une roue à main (36) qui est arrêtable dans différentes positions.

13. Dispositif selon la revendication 12, caractérisé en ce que quatre positions d'arrêt de la roue à main (3) sont prévues, à savoir une position de repos "0", une première position de travail "1" dans laquelle le disque d'impulsions (23) a pivoté dans le champ de mesure ; une deuxième position de travail "2" dans laquelle, supplémentairement au disque d'impulsion, une première zone (30) de l'aile en forme de couteau avec une faible masse est amenée à pivoter dans le champ de mesure ; et une troisième position de travail "3" dans laquelle, supplémentairement au disque d'impulsion, une deuxième zone (31) de l'aile avec une masse plus importante est amenée à pivoter dans le champ de mesure.

14. Dispositif selon l'une des revendications 4 à 13, caractérisé en ce que le capteur est formé par un organe de mesure capacitif (MK) et en ce que le disque d'impulsion (23) et l'aile (30, 31) sont réalisés en un matériau ayant une faible constante diélectrique d'une valeur de préférence non supérieure à 2,5.

15. Dispositif selon l'une des revendications 4 à 13, caractérisé en ce que le capteur est formé par un organe de mesure optique ou électro-optique.
